# EUROPEAN PATENT SPECIFICATION

(11) **EP 3 613 393 B1**
(45) Date of publication and mention of the grant of the patent: **10.11.2021**
(21) Application number: 19193370.4
(22) Date of filing: 23.08.2019
(51) Int. Cl.: A61F 13/15, B32B 3/10

(54) **PACKAGING MATERIAL**
VERPACKUNGSMATERIAL
MATERIAL D' EMBALLAGE

(30) Priority: 24.08.2018 US 201862722402 P
(43) Date of publication of application: 26.02.2020
(73) Proprietor: The Procter & Gamble Company, Cincinnati, OH 45202 (US)
(72) Inventor: Algers, John Daniel, Cincinnati, Ohio 45202 (US); Strasemeier, John Andrew, Cincinnati, Ohio 45202 (US); Gross, Sarah Beth, Cincinnati, Ohio 45202 (US)
(74) Representative: P&G Patent Germany

(56) References cited:
- WO-A1-2005/023530
- WO-A1-2015/041928
- WO-A1-2016/073686
- US-A1- 2004 122 395
- US-A1- 2006 025 736
- US-A1- 2006 025 737
- US-A1- 2011 045 252
- US-A1- 2017 285 474

## Description

### FIELD OF THE INVENTION

The present invention relates to a packaging material.

### BACKGROUND

Web materials and substrates, such as polymeric films, can be used as packaging and/or labels for consumer goods like disposable absorbent articles. Such substrates can also be suitable as components of these articles, including topsheets or backsheets.

It has been proposed to provide microtexture on substrates to enhance feel and appearance. Manufacturers often include printed graphics and/or text on products or packaging as well, but doing so in the presence of microtexture can lead to undesirable results. For example, microtextured substrates can be difficult to print on depending on the structural aspects of the microtexture and the chosen printing technique. The appearance of printed graphics and/or text can also be muted or distorted by the microtexture. Further, the printed text may not be sufficiently legible in the presence of microtexture, which is undesirable when the text is necessary to communicate important information such as safety precautions about the package contents.

Examples of conventional packaging materials in the form of textured laminate structures are provided in WO2015/041928-A1, for instance.

Thus, there is a need for a substrate having a desirable feel and containing printed artwork and/or text that are not negatively affected by inclusion of microtexture proximate to the printed portions. In addition, there is a need to balance tactile feel with desired features of flexibility, impermeability and/or strength. It would also be desirable to manufacture textured substrates and materials in a cost efficient and effective manner.

### SUMMARY OF THE INVENTION

The invention relates to a packaging material, as defined in claim 1, comprising:
a. a substrate having a first surface and an opposing second surface, wherein at least a portion of the first surface forms an exterior face of the packaging material;
b. a printed portion that is viewable from the first surface;
c. wherein the first surface comprises a textured zone comprising a plurality of discrete three-dimensional projections;
d. wherein at least some of the plurality of discrete three-dimensional projections comprise a sidewall extending outwardly from the first surface and a distal end;
e.wherein there is at least some overlap between the textured zone and the printed portion; and
f. wherein the printed portion comprises a color having coordinates that are outside a CIELab color boundary described by the following equations:
   i. b^{∗} = ―4.77a^{∗} - 42.21, wherein a^{∗} = -10.14 to - 7.72 or wherein b^{∗} = 6.18 to - 5.39;
   ii. b^{∗} = ―0.32a^{∗} - 7.85, wherein a^{∗} = ―7.72 to 1.29 or wherein b^{∗} = ―5.39 to - 8.26;
   iii. b^{∗} = 0.82a^{∗} - 9.32, wherein a^{∗} = 1.29 to 9.36 or wherein b^{∗} = -8.26 to - 1.64;
   iv. b^{∗} = ―4.08a^{*} + 36.52, wherein a^{∗} = 6.59 to 9.36 or wherein b^{∗} = 9.63 to - 1.64;
   v. b^{∗} = ―0.44a^{∗} + 12.53, wherein a^{∗} = ―2.19 to 6.59 or wherein b^{∗} = 13.49 to 9.63; and
   vi. b^{∗} = 0.92a^{∗} + 15.51, wherein a^{∗} = ―10.14 to ― 2.19 or wherein b^{∗} = 6.18 to 13.49; and
   wherein an L^{∗} of the color is 100 or less.

Additionally, the printed portion may comprise a printed character having a P/C Ratio of 3 or greater and/or a N/A Ratio of 6 or greater.

Claim 15 defines a method of making a packaging material, according to any of claims 1 to 13, and comprises the steps of:
a) providing a substrate comprising a first surface, an opposing second surface, and a textured zone comprising a plurality of discrete three-dimensional projections comprising a sidewall extending outwardly from the first surface;
b) printing the first and/or the second opposing surfaces to create one or more printed portions, wherein at least some of the textured zone overlays at least some of at least one printed portion;
c) manipulating the textured zone in an area so that at least some of the plurality of discrete three-dimensional projections are physically altered to change the visible appearance of the printed portion in the area.

### BRIEF DESCRIPTION OF THE DRAWINGS

Fig. 1 is a perspective view of a substrate comprising a textured zone and a flat zone.
Fig. 2 is schematic view of a closed distal end texture projection and an open distal end texture projection.
Fig. 3 is a plan view of a substrate comprising a textured zone, a flat zone, and printed portions that overlap with each of these two zones.
Fig. 4 is a color space graph.
Fig. 5 is another color space graph.
Fig. 6 is graph representing a relationship between printed text size and center-to-center spacing of microtexture projections.
Fig. 6A is a schematic representation of a character on a textured zone.
Fig. 6B is another schematic representation of a character on a textured zone.
Fig. 7A-7C are photomicrographs of structured substrates showing microtexture projections overlapping printed text.
Fig. 8 is a perspective view of an exploded laminate comprising a structured substrate comprising a textured zone and a flat zone, and an additional material layer.
Fig. 9 is a perspective view of an exemplary package comprising a microtextured substrate described herein.
Fig. 10 is a perspective view of another exemplary package comprising a microtextured substrate described herein.
Fig. 11 is a perspective view of an exemplary absorbent article.
Figs. 12-14 are schematic representations showing manufacturing methods described herein.
Figs. 15A-15E are photographs of packaging.
Figs. 16-18 are color space graphs showing a^{∗} and b^{∗} values measured from inventive examples.

### DETAILED DESCRIPTION OF EXEMPLARY EMBODIMENTS

### Definitions

"Absorbent article" means a device that absorbs and contains body exudates and, more specifically, devices that are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Design element" as used herein means a shape or combination of shapes that visually create a distinct and discrete component, regardless of the size or orientation of the component. A design element can be present in one or more patterns. A design element can be present one or more times within one pattern. In one example, the same design element is present twice in one pattern ― the second instance of the design element is smaller than the first instance. One of skill in the art will recognize that alternative arrangements are also possible. Design elements can comprise insignia. Design elements and/or combinations of design elements can comprise characters, words and/or graphics such as flowers, butterflies, hearts, cartoon representations and the like. Design elements and/or combinations of design elements can comprise instructional indicia providing guidance or instruction to the caregiver relative to placement and/or fit of the article about the wearer.

"Disposable," in reference to articles, means that the articles are generally not intended to be laundered or otherwise restored or reused in the same capacity (i.e., they are intended to be discarded after a single use and, preferably, to be recycled, composted or otherwise discarded in an environmentally compatible manner).

"Film" means a sheet-like material wherein the length and width of the material far exceed the thickness of the material (e.g., 10x, 50x, or even 1000x or more). Films are typically liquid impermeable but can be configured to be breathable.

"Ink" as used herein includes one or more of pigments, toners, inks and/or dyes.

"Insignia" as used herein means objects; representations of people; cartoons; animals and the like; words; colors; shapes or other indicia that can be used to distinguish, identify or represent the manufacturer, retailer, distributor or brand of a product, including trademarks, logos, emblems, symbols, designs, figures, lettering, crests or similar identifying marks.

"Microtexture" as used herein means at least 30 discrete elements per linear inch, wherein each discrete element comprises a height in the z-direction of at least about 30 micrometers.

"Pattern" as used herein means a decorative or distinctive design, not necessarily repeating or imitative, including the following: clustered, geometric, spotted, helical, swirl, arrayed, textured, spiral, cycle, contoured, laced, tessellated, starburst, lobed, blocks, pleated, concave, convex, braided, tapered, and combinations thereof.

"Substrate" includes any material that can be printed on. Thus, substrates of the present invention include nonwovens, fibrous polyolefin webs, polymeric films, apertured films, cellulosic webs, laminates of one or more of the above or any combination of one or more of the above. Substrates can be formed from webs and/or can comprise webs.

"Visible" as used herein means capable of being seen by a person having 20/20 vision from a distance of at least 12 inches away, under the unimpeded light of an ordinary incandescent 60 watt light bulb that is inserted in a fixture such as a table lamp.

"Web" or "web material" means a material capable of being wound into a roll. Webs can be films, nonwovens, laminates, apertured films and/or laminates, and the like.

### Description with Reference to the Figures

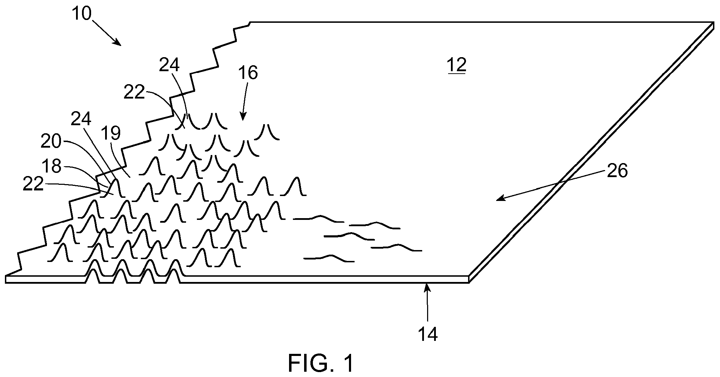

As shown in Fig. 1, a substrate 10 comprises a first surface 12 and an opposing second surface 14. The substrate 10 may comprise a film. The substrate can be monolayer or multilayer, such as a single layer film or a multilayer film. The substrate comprises polymeric materials, including polyethylene and/or polypropylene. Other synthetic and/or natural materials can be employed. The substrate can comprise a basis weight of at least about 14 gsm (grams per square meter), or at least about 17 gsm, or at least about 20 gsm, or at least about 22 gsm, or at least about 25 gsm, or from about 14 to about 100 gsm, reciting for said range every 2 gsm increment therein.

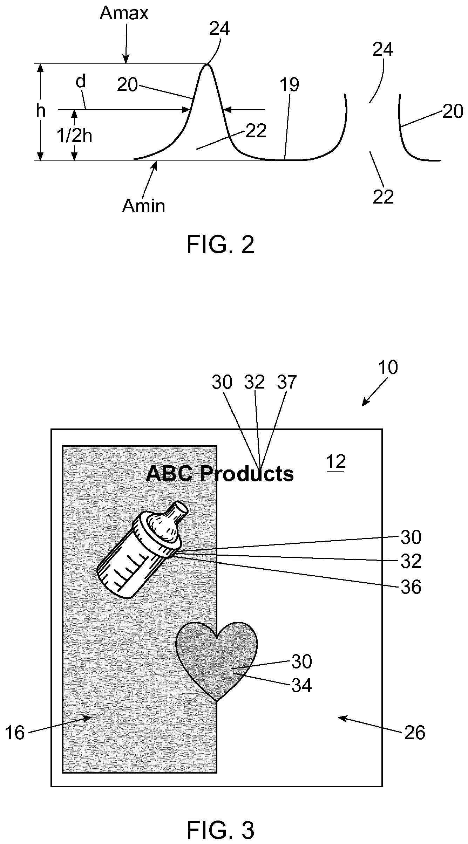

The substrate comprises at least one textured zone 16 that comprises a plurality of discrete projections 18 and land areas 19 therebetween. The discrete projections extend from first surface 12 (i.e., extend above the microplane of the web from the first surface) as is schematically depicted in Figs. 1 and 2. The density of the discrete projections can vary. For example, the textured zone can include at least 30, 60, 70, 90, or 150 discrete projections per linear inch. A suitable density range for projections 18 is from about 30 to about 120 per linear inch. Textured zone 16 can occupy substantially the entire first surface 12 or just a portion thereof. For example, at least about 40%, 50%, 75%, 90%, 95% of the area of first surface 12 comprises projections 18.

Textured zones can be produced by a variety of processes, including those disclosed in U.S. Patent No. 7,402,723. Projections 18 are formed in polymeric substrates by permanent local plastic deformation of the web. As shown in Fig. 2, projections 18 comprise a side wall(s) 20 extending from an open proximal portion 22 to a closed or open distal end 24. Textured zone 16 can include a combination of projections with some of the projections having a closed distal end 24 and others having an open distal end 24. Land areas 19 are portions of the substrate that are not substantially deformed and surround the proximal portions 22 of projections 18.

With reference again to Fig. 2, projections 18 have a height h measured from a minimum amplitude Amin between adjacent elements to a maximum amplitude Amax at the closed or open distal end 24. Textured zone 16 will typically have projections of varying heights, with the average height suitably in the range of from about 30, 40, 50, or 60 micrometers to about 400 micrometers. One of ordinary skill the art should appreciate that projections having different heights than in the preceding numerical range could be used without departing from the scope of the invention, defined in the appended claims 1-15.

Projections 18 have a diameter d, which for a generally cylindrical structure is the outside diameter cross-section generally parallel to the plane of the first surface 12. For projections having non-uniform lateral cross-sections, and/or non-cylindrical structures of projections 18, diameter d is measured as the average lateral cross-sectional dimension at half the height h of the discrete element, as shown in Fig. 2. Similar to the height discussion above, textured zone 16 will typically have projections of varying effective diameters, with the average effective diameter suitably in the range of from about 100 micrometers to about 400 micrometers. Projections having effective diameters outside of this numerical range can also be employed.

An aspect ratio, defined as h/d, can be determined for the discrete projections. The discrete projections can have an aspect ratio h/d of at least about 0.2, at least about 0.3, at least about 0.5, at least about 0.75, at least about 1, at least about 1.5, or at least about 2.

As noted above, textured zone 16 may not occupy all of first surface 12 of substrate 10 such that one or more flat zones 26 (shown in Figs. 1 and 3) exist on the substrate. A flat zone 26 is a portion of the substrate that contains less than 20, 10, 5, or 1 projections 18 per square inch area. The flat zone can for example be at least about 0.035 square inches (0.226 square cm). The flat zone is a portion of the substrate that is not deformed to include discrete projections or a portion of the substrate that initially included projections but have subsequently been altered. For example, the projections can have been collapsed with an applied force so that the projections have a height of less than 30 micrometers. In some examples, the projections may be compressed through an embossing process.

With reference to Fig. 3, the substrate can further comprise one or more printed portions 30. Fig. 3 represents a substrate 10 having a textured zone 16 and a flat zone 26. Although the textured zone 16 is shown in various figures herein as having a visually-noticeable texture for illustration purposes, microtexture can be difficult to discern visually, or not visible, on a manufactured good or packaging.

The printed portion can be disposed in the textured zone and/or in the flat zone. The printed portion can comprise one or more inks 32. The ink can comprise cyan, magenta, yellow, black or combinations thereof. Other inks and combinations can also be employed for the printed portion. The printed portion can be provided by various printing methods, such as flexographic printing, rotogravure printing, screen-printing, inkjet printing (including digital inkjet printing), cold laser printing and the like. Suitable inks for printing include water-based, solvent-based, and energy curable inks. Exemplary inkjet printing processes are described in U.S. Patent Nos. 3,465,350; 3,465,351; and 9,211,356.

Ink 32 can be applied in different amounts according to desired appearance and effects, as well as chosen applications of the printed substrate. By way of example only, applied ink 32 can be applied to have a basis weight of from about may have a basis weight of at least about 0.5 gsm, or at least about 1.0 gsm, or at least about 1.5 gsm, or from about 1 gsm to about 7 gsm, or about 1.5 gsm to about 5.5 gsm, or about 6 gsm or less, reciting for said ranges every 0.5 gsm increment therein. The printed portion 30 can have a print resolution of from about 10 dpi (dot per inch) to about 6000 dpi, including specifically every 10 dpi increment therein.

The printed portion can comprise a pattern. In some examples, however, the ink is disposed such that there is no discernable pattern in the printed portion. The ink(s) can be disposed on the substrate to include design elements 34, including insignia, characters, and words. The design elements can be in the form of one or more graphics 36, including images of flowers, butterflies, hearts, cartoon representations and the like.

The printing can occur on either or both surfaces 12 and 14, but it can be advantageous to print on the surface 14 opposite to that of the projections. When printing occurs on the surface 12 from which the projections extend, ink cannot be disposed completely or uniformly on the side walls of the discrete projections. As illustrated in Fig. 3, the printed portions can reside in a textured zone 16, a non-textured or flat zone 26, or both.

Texturing, especially microtexturing, can cause the overlapped printed portion to be muted, distorted, illegible, or the like. Without being bound by theory, it is believed that a printed portion is not sufficiently vibrant if all of its color coordinates are within certain color spaces defined by the CIELab L^{∗}a^{∗}b^{∗} color space. Indeed, the inventors have discovered that printed portions on substrate surface 12 from which the projections extend having a totality of coordinates defined within the below set of equations provide muted, dull and/or cloudy images that are unsatisfactory to product purchasers.
a) b^{∗} = ―4.77a^{∗} - 42.21, wherein a^{∗} = ―10.14 to ― 7.72 or wherein b^{∗} = 6.18 to - 5.39;
b) b^{∗} = ―0.32a^{∗} - 7.85, wherein a^{∗} = ―7.72 to 1.29 or wherein b^{∗} = ―5.39 to - 8.26;
c) b^{∗} = 0.82a^{∗} - 9.32, wherein a^{∗} = 1.29 to 9.36 or wherein b^{∗} = ―8.26 to - 1.64;
d) b^{∗} = ―4.08a^{∗} + 36.52, wherein a^{∗} = 9.36 to 6.59 or wherein b^{∗} = ―1.64 to 9.63;
e) b^{∗} = ―0.44a^{∗} + 12.53, wherein a^{∗} = 6.59 to - 2.19 or wherein b^{∗} = 9.63 to 13.49; and
f) b^{∗} = 0.92a^{∗} + 15.51, wherein a^{∗} = ―10.14 to - 2.19 or wherein b^{∗} = 1349 to 6.18.

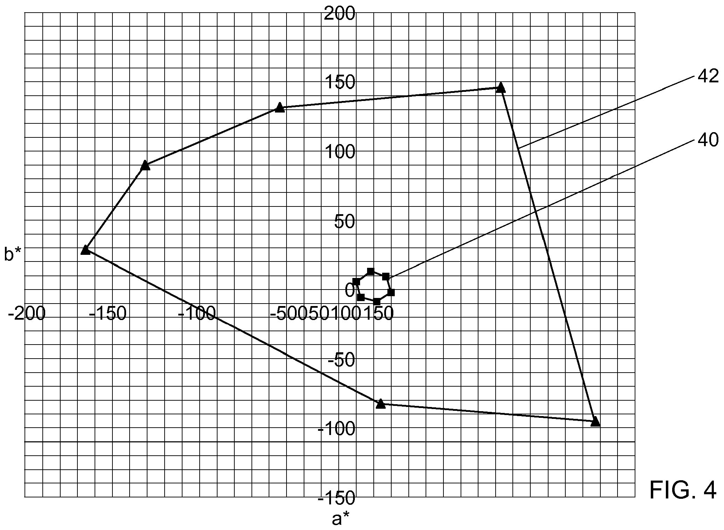

The boundary 40 of the space defined by these equations is shown in Fig. 4, along with broader CIELab space boundary 42. Thus, where a printed portion is disposed on a surface of the substrate within, or partially within, the textured zone (surface 12), the printed portion can comprise one or more a^{∗} and b^{∗} coordinates, or a majority of a^{∗} and b^{∗} coordinates, defined outside of the preceding system of equations (i.e., between boundary 40 and 42). The L^{∗} value can vary without significant effect on the vibrancy provided CIELab the a^{∗} and b^{∗} coordinates are defined outside of the preceding equations. A printed portion can comprise a L^{∗} value of at least about 50, or about 100 or less, or about 97 or less, or about 95 or less, or about 90 or less or about 85 or less, or from about 30 to about 100, or about 50 to about 97, reciting for each range every increment of 2 therein.

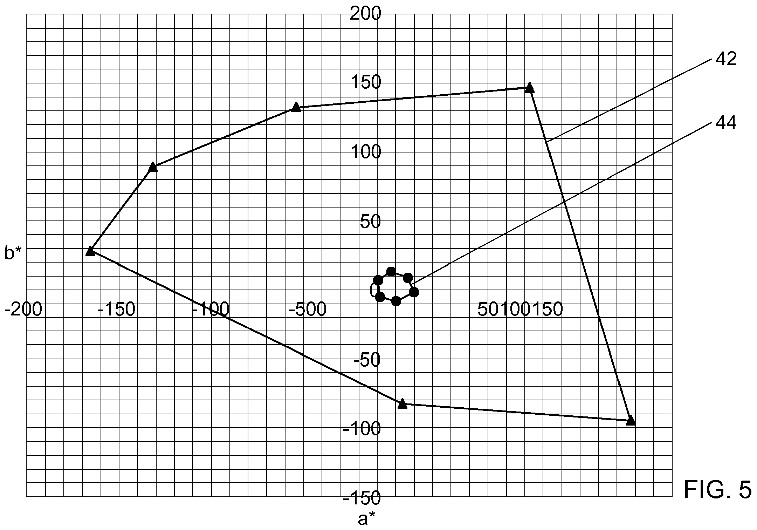

Turning to Fig. 5, the inventors have also discovered that printed portions disposed on the opposing surface 14 (i.e., the surface opposite of the surface 12 from which the discrete projections extend) and overlapping a textured zone that have a totality of coordinates within the following system of equations can provide muted, dull and/or cloudy images that are unsatisfactory to product purchasers:
a) b^{∗} = ―7.79a^{∗} - 70.16, wherein a^{∗} = ―9.84 to - 8.38 or wherein b^{∗} = 6.46 to - 4.88;
b) b^{∗} = ―0.38a^{∗} - 8.10, wherein a^{∗} = ―8.38 to 0.21 or wherein ; b^{∗} = ―4.88 to - 8.18;
c) b^{∗} = 0.68a^{∗} - 8.32, wherein a^{∗} = 0.21 to 9.78 or wherein b^{∗} = ―8.18 to ― 1.67;
d) b^{∗} = ―3.35a^{∗} + 31.10, wherein a^{∗} = 9.78 to 6.68 or wherein b^{∗} = ―1.67 to 8.72;
e) b^{∗} = ―0.48a^{∗} + 11.94, wherein a^{∗} = 6.68 to - 2.39 or wherein b^{∗} = 8.72 to 13.09; and
f) b^{∗} = 0.89a^{∗} + 15.22, wherein a^{∗} = ―2.39 to - 9.84 or wherein b^{∗} = 13.09 to 6.46.

The boundary 44 for the space defined by these equations is shown in Fig. 5, along with broader boundary 42 defining the CIELab space. Thus, where a printed portion is disposed on the second surface 14 and at least partially overlapping the textured zone, the printed portion can comprise one or more a^{∗} and b^{∗} coordinates, or a majority of a^{∗} and b^{∗} coordinates, defined outside of the preceding system of equations (i.e., coordinates between boundaries 42 and 44). The L^{∗} value can vary without significant effect on the vibrancy provided the CIELab a^{∗} and b^{∗} coordinates are defined outside of the preceding equations. Suitable L^{∗} values include those mentioned above in connection with Fig. 4.

Additionally, or alternatively, the printed portion may comprise a ΔE^{∗} value of at least about 2, or at least about 5, or at least about 6, or at least about 10, or at least about 15, or at least about 20, or from about 2 to about 60, or about 6 to about 50, reciting for each range every 1 increment therein.

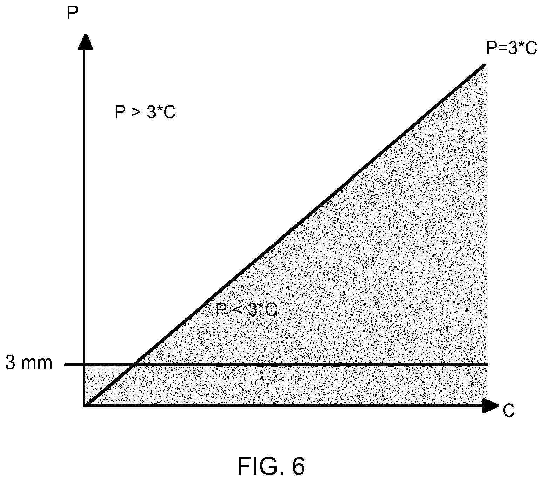

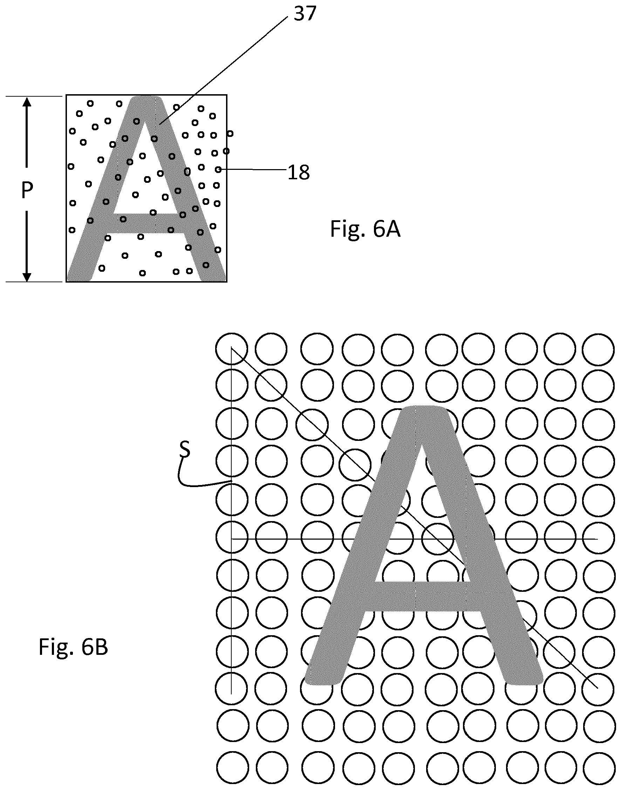

As shown in Fig. 3, a printed portion 30 that overlaps, at least in part, a textured zone 16 can contain text having one or more characters 37. The plurality of projections defining the texturing can make it difficult to read the text, particularly where smaller font height sizes, such as 11 pt font or less (i.e., 0.152 inches or less), are used for the characters. The inventors have discovered that managing characteristics of the microtexture projections and/or font size height of a character 37 can lead to printing overlapped by microtexture that generally is legible. In some examples, a printed portion comprises a ratio of font size height to the center-to-center spacing between adjacent projections (a "P/C Ratio" hereinafter) that is greater than 3, which leads to printing overlapped by microtexture that generally is legible. The P/C Ratio may be determined by the Character Measurement Test Method herein. The P/C Ratio may be at least 3, or at least 4, or at least 6. Fig. 6 is a graph that illustrates this relationship, wherein the upper left portion of the graph represents font size and center-to-center projection spacing that leads to legible text printing and the lower right shaded portion of the graph represents printing and texture choices that yield printed text that is less legible or even illegible. It is to be recognized that font height less than 3 mm is not typically legible as indicated by the shading in the chart. Figs. 6A schematically depicts the measurement of the font size height, P, of the character, and Fig. 6B schematically depicts how to determine center-to-center spacing for projections in or proximate to the printed character as is explained in the Character Measurement Test Method herein.

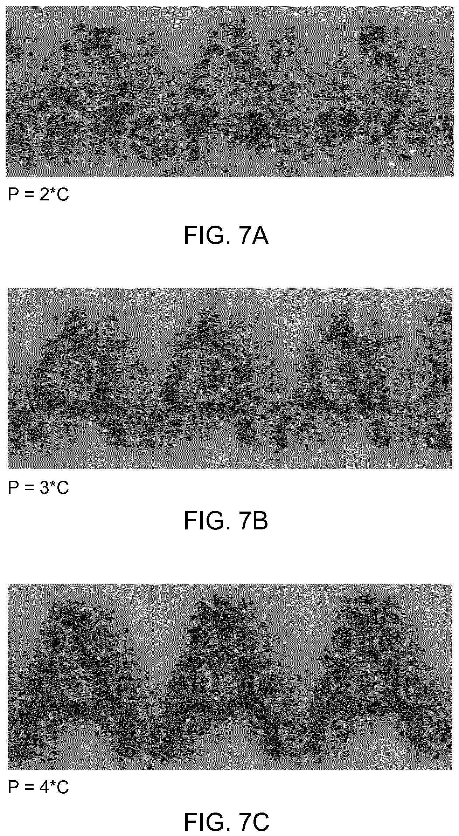

Figs. 7A-7C show different text heights as viewed through a 60 mesh textured film (i.e., a film having 60 discrete projections per linear inch). Fig. 7A shows printed text wherein a P/C Ratio is 2. The printed text in Fig. 7A is illegible. Fig. 7B shows printed text wherein the P/C Ratio is 3, and illustrates this is the border of where printed text becomes somewhat legible. Lastly, 7C printed text wherein the P/C Ratio is 4, wherein the printed letter "A" is visually discernable.

The printed portion may comprise a N/A Ratio of at least 4, or at least 6, or at least 20, or at least 30, or from about 5 to about 200, or from about 20 to about 100 reciting for each range every 5 increment therein, as determined by the Character Measurement Test Method herein.

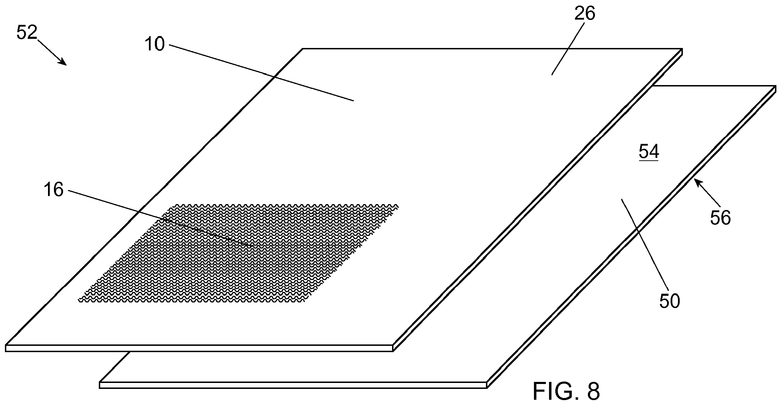

Substrate 10 can be joined to one or more additional material layers. By way of example and with reference to Fig. 8, substrate 10 can be joined to material layer 50 to form a laminate 52. Various techniques can be employed to form the laminate, including adhesively or ultrasonically joining substrate 10 and material layer 50. The material layer 50 can be used to provide greater water resistance, impermeability, strength, integrity or other features that the substrate 10 may lack. Material layer 50 can comprise any suitable material, including a nonwoven, a film, or combinations thereof. Material layer 50 can be void of microtexture. The material layer comprises a proximate side 54 and a distal side 56, wherein the proximate side 54 is joined in facing relationship to the substrate 10. The additional material layer can however comprise embossing or other macrotextures. Material layer 50 can comprise a basis weight in the range of from about 5 gsm to about 100 gsm. While not shown, laminates of the present invention can include three or more material layers with at least one of them comprising microtexture. Laminates can include multiple layers wherein both outer surfaces of the laminate contain a microtexture zone, wherein the plurality of discrete projections are the same or different from one another, so that a desired feel can be achieved from both sides of the laminate.

The printed portion(s) can be disposed on any surface of the laminate layers, including the first surface 12 which may comprise microtexture, the second opposing surface 14, the proximate surface 54 or the distal surface 56. The printed portion, or at least parts of the printed portion, may be visible from an exterior surface of the laminate, including an exterior surface comprising microtexture. In certain examples, one or both of the interior surfaces of a laminate comprise a printed portion. For example, printed portions can be disposed on surface 14 of substrate 10 and/or surface 54 of material layer 50. The printing accordingly is conducted prior to laminating the two material layers together. An exemplary laminate includes a first film layer comprising a textured zone comprising a plurality of discrete projections extending from an outer laminate surface, and a second film layer comprising printed portions on its surface that faces the first film layer. The characteristics of the first film layer are such that the printed portions on the second film layer are visible when viewing the laminate from the first film side. For example, the first film comprising the microtexture can be clear/transparent or contain less than 2%, 1%, 0.8%, or 0.5%, by weight of the first film, of pigment. Colored films are also contemplated herein, including, for example, white films that can have pigments at incorporation levels of greater than 2%.

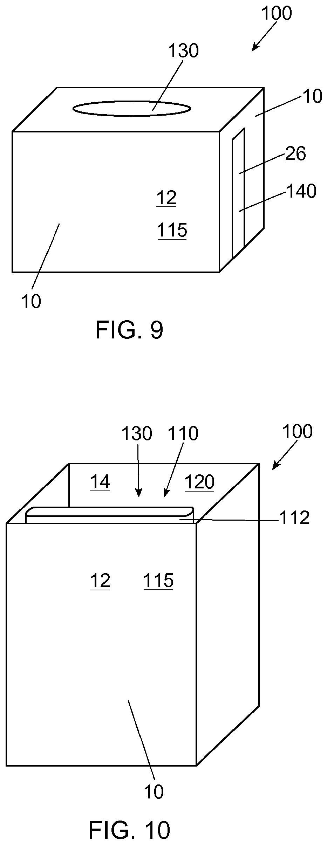

Substrates and laminates of the present invention can be employed for packages that contain one or more consumer products, including, for example, disposable absorbent articles, nonwoven wipes, or sanitary tissue products. The substrate/laminate can comprise any of the features described above, including a textured zone, flat zone, printed portion and any combination thereof. With reference to Figs. 9 and 10 ,a package 100 includes an interior portion 110 to contain one or more products 112. The substrate or laminate can be folded or otherwise manipulated to provide multiple sides of package 100 as shown in Figs. 9 and 10. The first surface 12 of the substrate or laminate can form an external face 115 of the package, and the second surface of the substrate or laminate can form an internal surface 120 of the package. The package can comprise an opening 130 and can otherwise be substantially closed such that enclosed articles are substantially surrounded by the packaging material. Alternatively, the package can be in the form of a bag such that the opening 130 is essentially the size of a side (see Fig. 10). In some examples, the package may include a window 140 such that the interior of the package may be viewed through the window. The window may be transparent or semi-transparent. Any suitable package configuration is possible, as long as it is within the scope of the present invention, as defined in the appended claims. Packages may comprise a substrate or laminate as described herein for part of its packaging in combination with other packaging materials that are different from the microtextured substrate/laminate material.

The substrate 10 may comprise a label, sticker or the like. The substrate 10 may in some examples comprise an adhesive on one or more sides, such that the substrate may be joined to another component. For instance, a substrate 10 may comprise an adhesive label such that the substrate can be affixed to a package or product. Microtexture may be disposed on the exterior surface of the label or sticker.

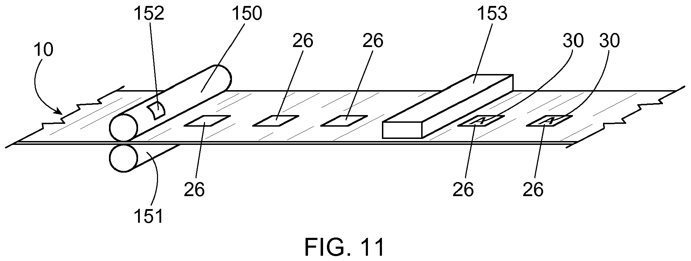

The substrate 10 may form a portion of an absorbent article 200, and microtexture may be disposed an exterior portion of an absorbent article. The absorbent article may be disposable. The absorbent article may be in the form of a taped diaper, a pant, or a feminine hygiene article. As shown in Fig. 11, the absorbent article 200 may comprise a topsheet 224, backsheet 226 and absorbent core disposed between the topsheet and backsheet. It should be recognized that other structures, elements, or substrates may be positioned between the core and the topsheet 224 and/or backsheet 226, including an acquisition-distribution system. In certain examples at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. Among other benefits, it is believed that when at least a portion of the absorbent core is substantially cellulose free, this portion of the absorbent core is significantly thinner and more flexible than a similar absorbent core that includes more than 10% by weight of cellulosic fibers. The core may comprise one or more channels, which are substantially free of absorbent material. In one example, one or more channels may extend longitudinally. While the topsheet 224, the backsheet 226, and the absorbent core 228 may be assembled in a variety of well-known configurations, absorbent article configurations are described generally in U.S. Patent Nos. 3,860,003; 5,151,092; 5,221,274; 5,554,145; 5,569,234; 5,580,411; 6,004,306; 8,979,815; and 9,060,904, and U.S. Pat. App. Nos. 14/598,783 and 14/032,595. Any of the following article components may comprise a substrate 10, having any of the features described above: the topsheet 224, backsheet 226, leg gasketing system 270 (including barrier 271 and/or gasketing cuffs 272), fastening system 242, ear 240, landing zone 230, waist feature 280, such as an elasticized waistband and/or belt.

A printed substrate or laminate of the present invention can be made by providing a web with a first and second surface as described above. A plurality of discrete projections can be provided to extend from the first surface forming a textured zone. The discrete projections can be provided by processes disclosed in U.S. Patent No. 7,402,723, for example, and can have any of the features described above. In some examples the discrete projections are formed by vacuum aperturing. The textured zone can comprise microtexture. The substrate can further include a flat zone 26. The flat zone can be provided by refraining from forming discrete projections in one or more areas of the substrate. The flat zone can be also provided by manipulating (for example via pressure and/or heat) the discrete projections in an area of the textured zone so that at least some of the plurality of projections are physically altered to change the visual appearance of printed portion(s) within the manipulated area. The discrete projections may be reduced in height, or eliminated by the manipulation.

The printed portion can be provided on the substrate or laminate via any suitable printing technique, including flexographic printing, inkjet printing, laser printing and combinations thereof. The printed portion can include a small font (6, 8, 10, or 12 font, for example) character. Printing can occur before or after forming discrete projections in the web. Printing may be provided on any suitable surface of the laminate, as described above. Printing may at least partially overlap a textured zone and/or a flat zone. The manipulation of some of the projections to effect a flat zone can occur before or after printing. And lamination of a substrate comprising microtexture to one or more additional material layers can occur before or after the manipulation of some of the projections to effect a flat zone in the substrate comprising microtexture.

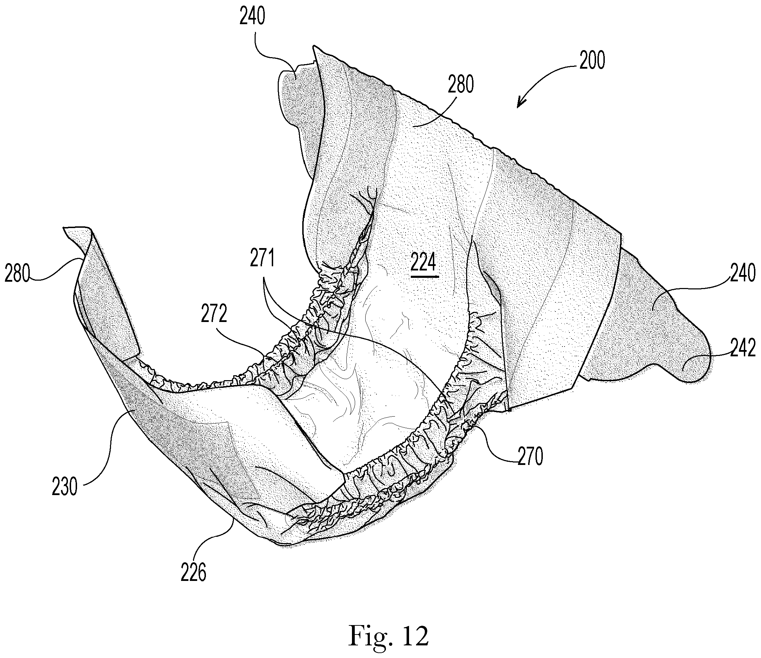

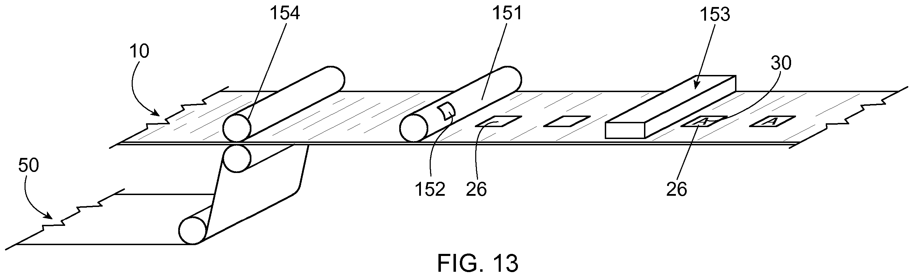

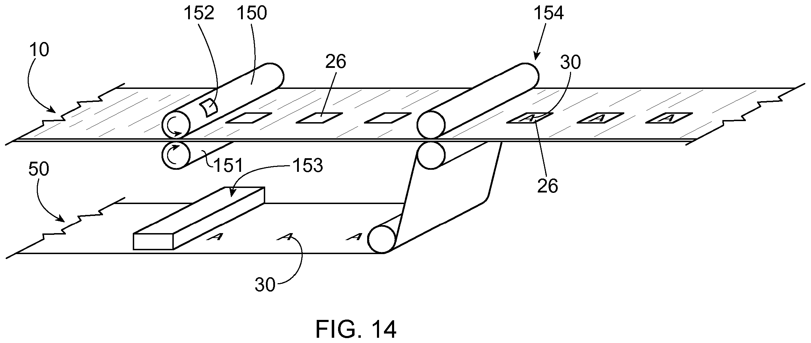

Figs. 12-14 illustrate exemplary methods contemplated herein. Fig. 12 shows a single layer structured substrate 10 comprising texture that is selectively embossed via a pair of embossing rolls 150 and 151, with embossing roll 151 having a raised portion 152 that alters texture projections to effectively create flat zones 26 in substrate 10. Thereafter, substrate 10 passes under a print head 153 that creates a printed portion 30 on substrate 10 in at least flat zone 26. Note that the method shown in Fig. 12 prints on the film substrate surface from which the textured projections extend. The substrate can alternatively or additionally be printed on its opposite surface such that the printing is at least partially within flat zone 26. Figs. 13 and 14 show methods for making a laminate as contemplated herein. A non-textured film 50 is laminated to structured film substrate 10 prior to printing and creating a flat zone, using a lamination device 154. The lamination can occur via adhesives or through non-chemical techniques such as application of heat and pressure or use of ultrasonic energy. Laminate 52 is selectively embossed by raised portion 152 on embossing roll 151 to create flat zones 26, and thereafter the laminate 52 is printed on via print head 153 so that printed portions 30 are registered with the flat zones 26. Fig. 13 shows non-textured film 50 that is printed on via print head 153 while a flat zone 26 is created on structured substrate 10, with film 50 and structured substrate 10 being laminated thereafter so that the printed portion 30 and the flat zone 26 are registered or overlap at least in part.

### Examples

Various designs combining printing and microtextured substrates/laminates can be achieved with the principles disclosed herein.

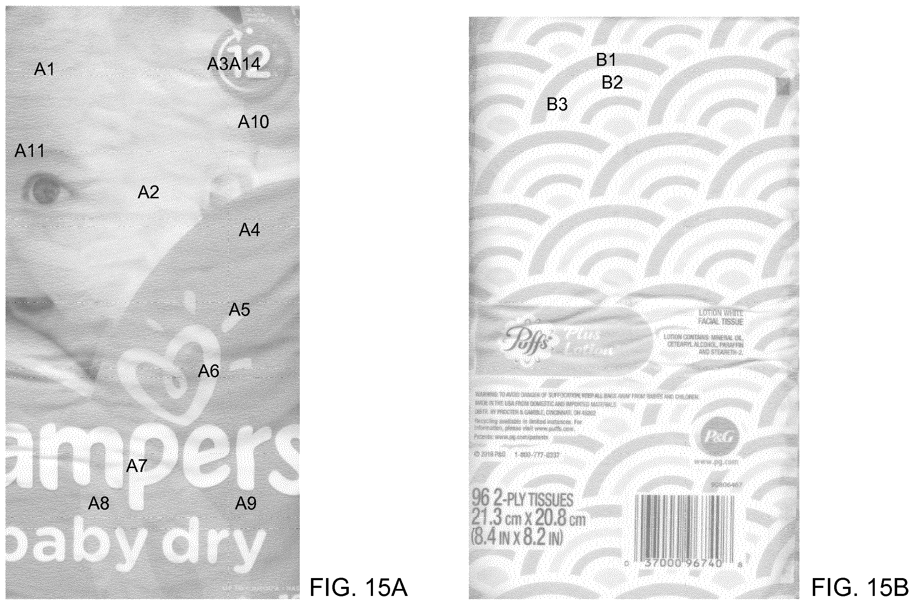

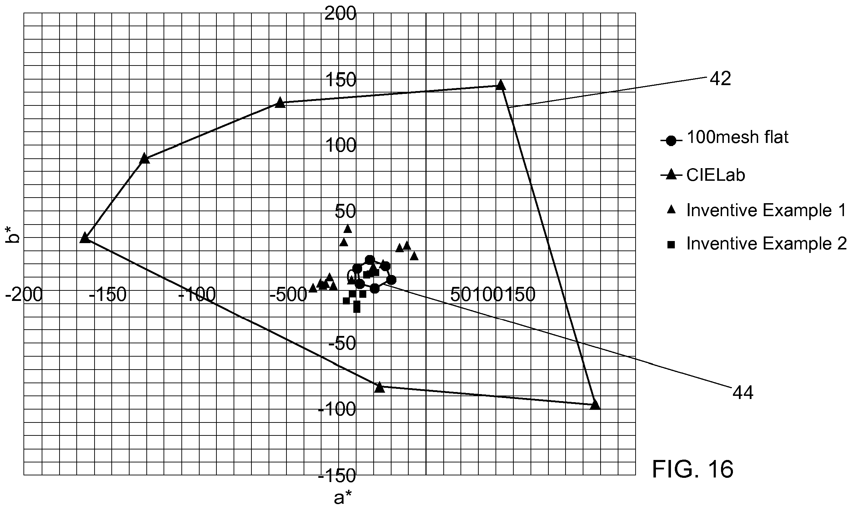

Example 1 comprises a single layer, clear, polyethylene film, having a basis weight of 25 gsm, and a first textured surface and a second opposing surface. The film is vacuum formed to create a plurality of projections having open distal ends and present in the film at 60 mesh. The discrete projections extend from the first film surface in a textured zone. The film is printed on the second opposing surface via inkjet printing. The print overlaps the textured zone of the film. The printed portion is shown in Fig. 15A. Measurements, shown in Table 1A, were taken in fourteen different areas. The measurements are taken from the first film surface (i.e., textured side) in accordance with the Color Measurement Test Method herein at 1200 dpi. Delta E shown in Table 1B are calculated between various adjacent areas in accordance with the Color Measurement Test Method herein. As can be seen from Table 1A and Fig. 16, the color values of a majority of the areas measured outside of boundary 44 (i.e., the boundary of the nonvibrant artwork on the second web surface).

**Table 1A**

| Area | Area size (pixels) | L^{∗} | a^{∗} | b^{∗} | Outside of Boundary 44 |
|---|---|---|---|---|---|
| A1 (Forehead) | 101×101 | 87.88 | 14.88 | 22.75 | Yes |
| A2 (Side of Head) | 101×101 | 97.38 | 5.25 | 9.50 | On border |
| A3 (Circle indicating wear time) | 101×101 | 89.75 | -25.38 | -0.25 | Yes |
| A4 (Background Section 1) | 101×101 | 88.75 | -29.13 | -6.75 | Yes |
| A5 (Heart Claws) | 101×101 | 88.63 | -27.88 | -5.25 | Yes |
| A6 (Heart) | 101×101 | 92.75 | -17.50 | 26.25 | Yes |
| A7 (Pampers) | 101×101 | 95.38 | -12.63 | -2.63 | Yes |
| A8 (Background Heart) | 101×101 | 91.13 | -23.38 | -6.50 | Yes |
| A9 (Background Section 2) | 101×101 | 85.25 | -34.88 | -8.14 | Yes |
| A10 (Background Section 3) | 101×101 | 91.75 | -15.25 | 36.63 | Yes |
| A11 (Eyebrow) | 101×101 | 82.63 | 18.75 | 23.75 | Yes |
| A12 (Eye -not pupil) | 101×101 | 67.88 | -0.38 | 7.50 | No |
| A13 (Lip) | 101×101 | 71.75 | 23.00 | 15.88 | Yes |
| A14 (small circles within larger circle) | 101×101 | 87.20 | -30.60 | -4.80 | Yes |

**Table 1B**

| Areas of Comparison | ΔE^{∗} |
|---|---|
| A1, A2 | 18.93 |
| A3, A14 | 7.38 |
| A8, A9 | 13.02 |
| A1, A11 | 6.60 |
| A4, A5 | 1.96 |
| A7, A8 | 12.19 |
| A1, A13 | 19.32 |
| A5, A9 | 8.29 |
| A2, A12 | 30.10 |

Example 2 comprises a laminate comprising a first film thermally embossed to a second film. The first film is a clear, polyethylene film, having a basis weight of 25 gsm and a first textured surface and a second opposing surface. The first film is vacuum formed with projections having open distal ends and present in the film at 60 mesh. The discrete projections extend from the first film surface in a textured zone. The first film is printed on its second surface via inkjet printing prior to lamination to the second film. The printed portions overlap the textured zone of the film. The second film surface is proximate to the second film during lamination, such that the first film surface forms an external surface of the laminate. The second film is clear and has a basis weight of 32gsm. The printed portion is shown in Fig. 15B. Measurements, shown in Table 2A, were taken in 8 different areas of the printed portion. The measurements are taken from the first laminate surface (i.e., textured side) in accordance with the Color Measurement Test Method herein at 1200 dpi. Delta E shown in Table 2B are calculated between various adjacent areas in accordance with the Color Measurement Test Method herein. As can be seen from Table 2A and Fig. 16, the color values of a majority of the areas measured outside of boundary 44 (i.e., the boundary of the nonvibrant artwork on the second web surface). In addition, high ΔE^{∗} values were obtained.

**Table 2A**

| Area | Area Size (pixels) | L^{∗} | a^{∗} | b^{∗} | Outside of Boundary 44 |
|---|---|---|---|---|---|
| B1 (Arc 1) | 101×101 | 92.25 | 1.00 | 4.00 | No |
| B2 (Arc 2) | 101×101 | 98.00 | -4.13 | 2.00 | No |
| B3 (Base between Arcs) | 101×101 | 95.50 | -2.13 | 2.63 | No |
| B4 (Text) | 31×31 | 79.63 | -9.75 | -20.25 | Yes |
| B5 (Circle) | 101×101 | 84.63 | -15.88 | -17.50 | Yes |
| B6 (Text in Circle) | 31×31 | 89.63 | -12.38 | -12.50 | Yes |
| B7 (Bar Code) | 31×31 | 75.00 | -9.88 | -23.50 | Yes |
| B8 (Text 2) | 31×31 | 82.63 | -6.50 | -12.25 | Yes |

**Table 2B**

| Areas of Comparison | ΔE^{∗} |
|---|---|
| B1, B2 | 7.96 |
| B1, B3 | 4.71 |
| B2, B3 | 3.26 |
| B5, B6 | 7.89 |
| B3, B7 | 34.10 |
| B7, B8 | 14 |

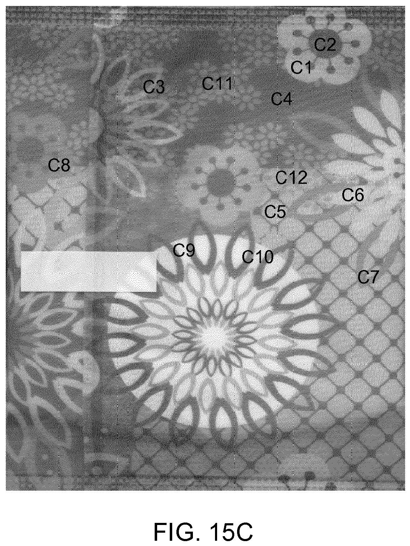

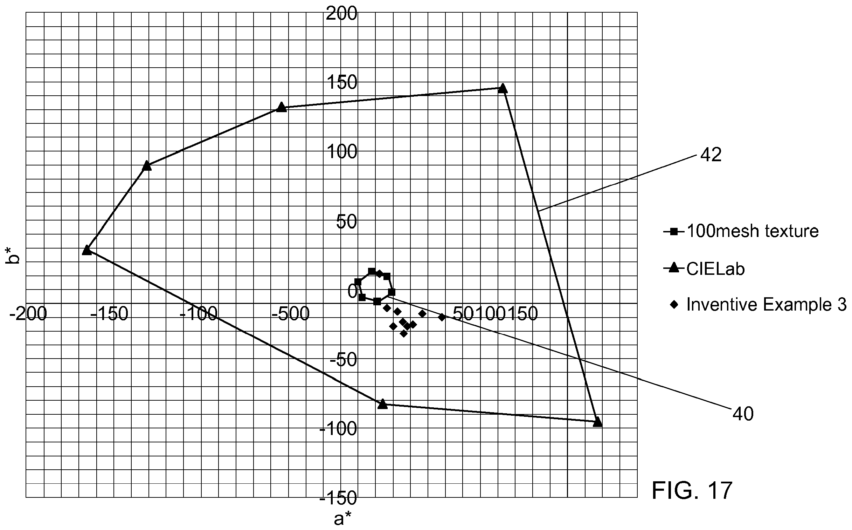

Example 3 comprises a laminate of a clear, polyethylene film, having a basis weight of 25 gsm, adhesively bonded to a 25 gsm nonwoven material. The film is vacuum formed with projections having open distal ends and present in the film at 60 mesh. The discrete projections extend from a first film surface in a textured zone. The film was printed on the first, textured web surface via inkjet printing. The print overlapped the textured zone of the film. The film's second surface is positioned proximate to the nonwoven material during lamination such that the first film surface forms an external surface of the laminate. The printed portion is shown in Fig. 15C. Measurements, shown in Table 3A, were taken in 12 different areas of the printed portion. The measurements were taken from the first web surface (i.e., textured side) in accordance with the Color Measurement Test Method herein at 1200 dpi. Delta E shown in Table 3B were calculated between various adjacent areas in accordance with the Color Measurement Test Method herein. As can be seen from Table 3A and Fig. 17, a majority of the areas measured outside of boundary 40 (i.e., the boundary of the nonvibrant artwork printed on the first web surface). In addition, high ΔE^{∗} values were obtained.

**Table 3A**

| Area | Area Size (pixels) | L^{∗} | a^{∗} | b^{∗} | Outside of Boundary 40 |
|---|---|---|---|---|---|
| C1 (Flower 1) | 101×101 | 58.13 | 2.63 | 11.63 | On border |
| C2 (Center of Flower 1) | 101×101 | 44.75 | 6.75 | -12.63 | Yes |
| C3 (Petals) | 51×51 | 49.38 | 10.50 | -26.25 | Yes |
| C4 (Background Section 1) | 101×101 | 41.75 | 16.88 | -31.63 | Yes |
| C5 (Diamonds Interior) | 101×101 | 61.88 | 16.00 | -22.63 | Yes |
| C6 (Petals 2) | 101×101 | 71.38 | 27.13 | -17.00 | Yes |
| C7 (Flower 2) | 101×101 | 62.88 | 38.25 | -19.25 | Yes |
| C8 (Flower 3) | 101×101 | 56.25 | 46.00 | -15.50 | Yes |
| C9 (White next to C10) | 11×11 | 72.75 | 12.88 | -15.43 | Yes |
| C10 (Petal 3) | 51×51 | 59.13 | 21.63 | -24.38 | Yes |
| C11 (Flower 4 on Background 1) | 51×51 | 51.13 | 16.00 | -31.38 | Yes |
| C12 (Flower 4 on Diamonds ) | 51×51 | 66.38 | 18.38 | -26.00 | Yes |

**Table 3B**

| Areas of Comparison | ΔE^{∗} |
|---|---|
| C1, C2 | 28.00 |
| C3, C4 | 11.30 |
| C5, C12 | 6.11 |
| C4, C11 | 9.42 |
| C6, C7 | 14.18 |
| C7, C8 | 10.86 |
| C9, C10 | 18.50 |

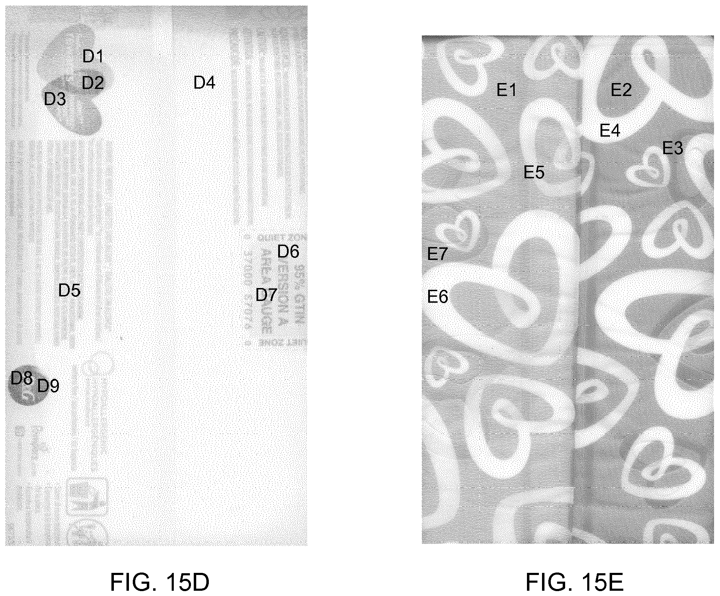

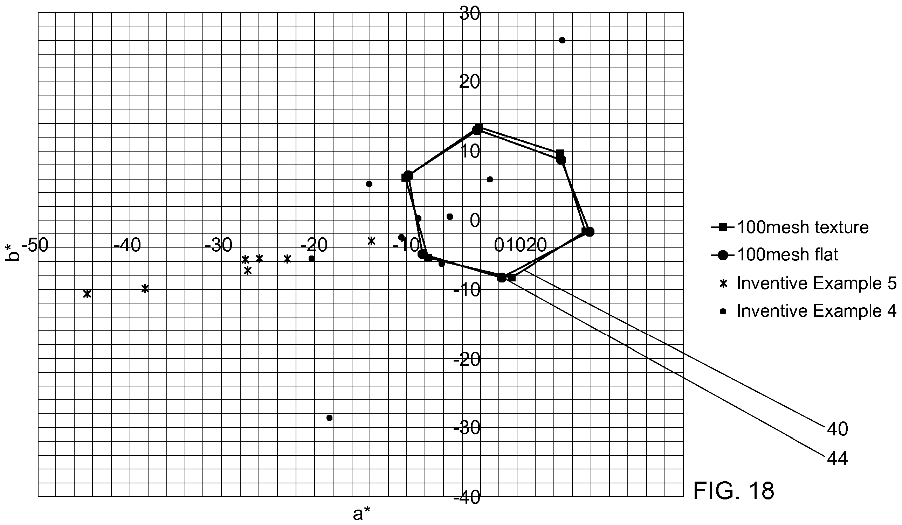

Example 4 comprises a laminate comprising a first film thermally embossed to a secondary film. The first film is a clear, polyethylene film, having a basis weight of 25 gsm and a first textured surface and a second opposing surface. The film is vacuum formed to create projections having open distal ends and present in the film at 60 mesh. The discrete projections extend from a first surface in a textured zone. The secondary film is a micro-embossed polyethylene film and has a basis weight of 32gsm and is a cast PE film. The secondary film was printed via injket printing prior to lamination. The printed surface of the secondary film is placed proximate to the second surface of the first film during lamination, such that the first surface forms an external surface of the laminate and the non-printed surface of the secondary film forms the other external surface of the laminate. During lamination, the films are arranged such that the printed portion overlaps the textured zone. The printed portion is shown in Fig. 15D. Measurements, shown in Table 4A, were taken in 9 different areas of the printed portion. The measurements were taken from the first surface (i.e., textured side) in accordance with the Color Measurement Test Method herein at 1200 dpi. Delta E shown in Table 4B were calculated between various adjacent areas in accordance with the Color Measurement Test Method herein. As can be seen from Table 4A and Fig. 18, the color values of a majority of the areas measured outside of boundaries 40 (i.e., the boundary of nonvibrant artwork on the first web surface) and 44 (i.e., the boundary of the nonvibrant artwork on the second web surface). In addition, high ΔE^{∗} values were obtained.

**Table 4A**

| Area | Area Size (pixels) | L^{∗} | a^{∗} | b^{∗} | Outside of Boundary 40 | Outside of Boundary 44 |
|---|---|---|---|---|---|---|
| D1 (Heart 1) | 101×101 | 84.25 | -10.63 | -2.50 | Yes | Yes |
| D2 (Overlap between Heart 1 and Heart 2) | 101×101 | 77.88 | -14.13 | 5.25 | Yes | Yes |
| D3 (Heart 2) | 101×101 | 82.38 | 6.88 | 26.00 | Yes | Yes |
| D4 (Background) | 101×101 | 97.75 | -1.00 | 5.88 | No | No |
| D5 (Text 1) | 51×51 | 91.25 | -8.75 | 0.25 | No | No |
| D6 (Background of Text Box) | 51×51 | 92.13 | -5.38 | 0.50 | No | No |
| D7 (Text in Text Box) | 11×11 | 79.63 | -20.38 | -5.50 | Yes | Yes |
| D8 (Circle) | 51×51 | 67.00 | -18.50 | -28.50 | Yes | Yes |
| D9 (Text in Circle) | 11×11 | 85.13 | -6.25 | -6.29 | Yes | Yes |

**Table 4B**

| Areas of Comparison | ΔE^{∗} |
|---|---|
| D1, D2 | 18.93 |
| D1, D3 | 10.24 |
| D4, D5 | 11.57 |
| D8, D9 | 31.18 |
| D6, D7 | 20.43 |

Example 5 comprises a laminate comprising a first film thermally embossed to a secondary film. The first film is a clear, polyethylene film, having a basis weight of 25 gsm and a first textured surface and a second opposing surface. The film is vacuum formed to define projections having open distal ends and present in the film at 60 mesh. The discrete projections extend from a first surface in a textured zone. The secondary film is clear and has a basis weight of 32gsm and is a cast PE film. The secondary film was printed via inkjet printing prior to lamination. The printed surface of the secondary film is placed proximate to the second surface of the first film during lamination, such that the first surface forms an external surface of the laminate and the non-printed surface of the secondary film forms the other external surface of the laminate. During lamination, the films are arranged such that the printed portion overlaps the textured zone. The printed portion is shown in Fig. 15E. Measurements, shown in Table 5A, were taken in 7 different areas of the printed portion. The measurements were taken from the first web surface (i.e., textured side) in accordance with the Color Measurement Test Method herein at 1200 dpi. Delta E shown in Table 5B were calculated between various adjacent areas in accordance with the Color Measurement Test Method herein. As can be seen from Table 5A and Fig. 18, the color values of a majority of the areas measured outside of boundaries 40 (i.e., the boundary of nonvibrant artwork on the first web surface) and 44 (i.e., the boundary of the nonvibrant artwork on the second web surface). In addition, high ΔE^{∗} values were obtained.

**Table 5A**

| Area | Area Size (pixels) | L^{∗} | a^{∗} | b^{∗} | Outside of Boundary 40 | Outside of Boundary 44 |
|---|---|---|---|---|---|---|
| E1 (Background 1) | 101×101 | 91.25 | -27.50 | -5.75 | Yes | Yes |
| E2 (Background 2) | 101×101 | 88.63 | -38.38 | -9.88 | Yes | Yes |
| E3 (Embossed Area) | 101×101 | 86.13 | -44.63 | -10.63 | Yes | Yes |
| E4 (White Heart) | 101×101 | 93.38 | -23.00 | -5.63 | Yes | Yes |
| E5 (Brushed Heart) | 101×101 | 91.50 | -27.25 | -7.25 | Yes | Yes |
| E6 (2nd White Heart) | 101×101 | 96.63 | -13.88 | -3.00 | Yes | Yes |
| E7 (Lt Embossed) | 101×101 | 92.25 | -26.00 | -5.50 | Yes | Yes |

**Table 5B**

| Areas of Comparison | ΔE^{∗} |
|---|---|
| E1, E2 | 18.93 |
| E1, E5 | 51.84 |
| E6, E7 | 13.13 |
| E2, E5 | 11.79 |
| E2, E3 | 6.77 |
| E5, E7 | 2.28 |
| E3, E7 | 20.27 |

### Test Methods

### Image Preparation for Color Measurement and Character Measurement Test Methods

The image is collected and measurements are performed on the surface of a film from which the texture projections extend, regardless of whether the printing is on that surface or not. A flat-bed scanner capable of scanning 24-bit color at 1200 dpi and that has manual control of color management is used to acquire images. A suitable scanner is an Epson Perfection V750 Pro with Epson Scan software (Epson America Inc., Long Beach, CA), or equivalent. The scanner is calibrated against a color reflection target compliant to ANSI method IT8.7/2-1993 using color management software to construct a scanner color profile. Suitable software is i1Scanner for Epson (X-Rite, Grand Rapids, MI), or equivalent. The resulting calibrated scanner color profile is accessed with image editing software that supports sampling in CIE L^{∗} a^{∗} b^{∗} and used to measure color in an image of the textured surface of the film. Suitable software is Photoshop CS4 (Adobe Systems Inc., San Jose, CA), or equivalent.

The scanner is powered on for at least 30 minutes prior to calibration. The scanner is configured to collect images in 24-bit color and at 1200 dpi. Further, the scanner is set to collect in reflectance mode and with any unsharp mask or automatic color correction options deselected. If using Epson Scan software, this is achieved by for the Original options selecting Reflective mode as the Document Type, Document Table as the Document Source and Photo as the Auto Exposure Type. The unsharp mask setting and any automatic color correction or color management options are deselected. (As stated above, if the automatic color management cannot be disabled, the scanner is not appropriate for this method.)

An IT8 target is then placed squarely in the scanner, and an image of the target is acquired and subsequently cropped to exclude any white space around the target. Suitable color management software (such as XRite i1Scanner) is used to compare the IT8 target image with included reference files and to create and export a calibrated color profile compatible with image editing software such as Photoshop CS4.

A specimen is then placed in the scanner with the side from which the texture productions extend facing the glass (that is, facing the detector). The specimen is backed with a white background. In this test method, white background material is defined as exhibiting L^{∗} > 94, -2 < a^{∗} < 2, and -2 < b^{∗} < 2). A scanned, 24-bit color image is acquired using the same settings with which the image of the IT8 reference target was collected with the exception that lower spatial resolution, such as 600 dpi, may be used if desired.

### Color Measurement ― CIELAB

Color measurements are based on the CIE L^{∗} a^{∗} b^{∗} color system (CIELAB). Each sample image is prepared as above. The resulting image is imported into image editing software such as Photoshop CS4. The calibrated scanner color profile generated previously by color management software is assigned to the image, and the color mode is set to the option corresponding to CIE L^{∗} a^{∗} b^{∗}. (For example, in Photoshop CS4, "Lab Color" corresponds to the CIE L^{∗} a^{∗} b^{∗} color space.)

In the image editing software, a tool that enables local color sampling is used to interrogate the color properties of an area to be measured. (For example, in Photoshop CS4, the "eyedropper" tool is appropriate for this objective.) The sampling size of the tool, if adjustable, is set to include as many pixels as possible from the area to be measured in a single sampling. For each area to be measured, the local sampling tool is used to record eight individual L^{∗} a^{∗} b^{∗} values, each to the nearest 0.01 in value. The arithmetic mean of the eight individual L^{∗} a^{∗} b^{∗} values is calculated and recorded. This mean value is reported as the L^{∗} a^{∗} b^{∗} value of the area to be measured.

### Character Measurement Test Method

Prepare 4 substantially similar replicate samples for testing. Each sample image is prepared as above. Isolate a character by defining the smallest possible rectangular box that completely encloses the character about the outer perimeter of the character, with two sides generally perpendicular to the direction of the string of text (i.e., "length sides" hereinafter) and two sides perpendicular to the length sides (i.e., the "width sides" hereinafter). Where there is a single character in the image or the string of text does not follow a linear path/direction, the length sides are formed with lines that are generally parallel to the longest dimension of the character and the width sides are perpendicular to the length sides as shown in Fig. 6A. If there is a gradient of ink from the center of the character to the outer perimeter of the character, then the size of the box should be adjusted so that all of the ink is within the box. Commonly used software such as Microsoft Power Point and Photoshop can be used to draw the box.

### 1. Number per Character Area (N/A Ratio)

This testing is performed individually on four replicate samples. For each sample, mark the center of each discrete projection having a height of at least 30 micrometers that reside in or along a side of the box. Count the number centers identified and report it as N/A_{ind}. By way of example Fig. 6A has a N/ A_{ind} of 62. Calculate the arithmetic mean of N/A_{ind} values and record the average as the N/A Ratio.

### 2. Character Height per Spacing (P/C Ratio)

This testing is performed individually on four replicate samples. For each sample, measure the length side and record the value as P_{ind}, in mm to the nearest 0.1 mm.

To the extent that the box does not enclose at least 100 discrete projections, each having a height of 30-400 micrometers, extend the box such that the character is centered but at least 100 discrete projections are captured. Determine the shortest possible line, S, within the box that crosses the center of 10 discrete elements as shown in Fig. 6B. Record the length as L, in mm to the nearest 0.1 mm. Calculate the center-to-center distance, C_{ind}, as L/9.

Calculate the arithmetic mean of P_{ind} values and record the mean as P in mm to the nearest 0.1 mm. Calculate the arithmetic mean of C_{ind} values and record the mean as C in mm to the nearest 0.1 mm. P divided by C is recorded as the P/C Ratio.

## Claims

1. A packaging material (100) comprising:
a. a substrate having a first surface (12) and an opposing second surface (14), wherein at least a portion of the first surface forms an exterior face (115) of the packaging material;
b. a printed portion (30) that is viewable from the first surface;
c. wherein the first surface comprises a textured zone (16) comprising a plurality of discrete three-dimensional projections (18);
d. wherein at least some of the plurality of discrete three-dimensional projections comprise a sidewall (20) extending outwardly from the first surface and a distal end (24);
e. wherein there is at least some overlap between the textured zone and the printed portion;
**characterized in that**:
f. the printed portion comprises a color having coordinates that are outside a CIELab color boundary described by the following equations:
i) b^{∗} = ―4.77a^{∗} - 42.21, wherein a^{∗} = ―10.14 to ― 7.72 or wherein b^{∗} = 6.18 to - 5.39;
ii) b^{∗} = ―0.32a^{∗} - 7.85, wherein a^{∗} = ―7.72 to 1.29 or wherein b^{∗} = ―5.39 to - 8.26;
iii) b^{∗} = 0.82a^{∗} - 9.32, wherein a^{∗} = 1.29 to 9.36 or wherein b^{∗} = -8.26 to - 1.64;
iv) b^{∗} = ―4.08a^{∗} + 36.52, wherein a^{∗} = 6.59 to 9.36 or wherein b^{∗} = 9.63 to - 1.64;
v) b^{∗} = ―0.44a^{∗} + 12.53, wherein a^{∗} = ―2.19 to 6.59 or wherein b^{∗} = 13.49 to 9.63; and
vi) b^{∗} = 0.92a^{∗} + 15.51, wherein a^{∗} = ―10.14 to ― 2.19 or wherein b^{∗} = 6.18 to 13.49; and wherein an L^{∗} of the color is 100 or less.

2. A packaging material according to claim 1 wherein the substrate comprises a film.

3. A packaging material according to claims 1 or 2 wherein the substrate is printed on the second surface, and wherein the printed portion on the second surface comprises a color having coordinates that are outside a CIELab color boundary described by the following equations:
i. b^{∗} = ―7.79a^{∗} - 70.16, wherein a^{∗} = ―9.84 to - 8.38 or wherein b^{∗} = 6.46 to - 4.88;
ii. b^{∗} = ―0.38a^{∗} ― 8.10, wherein a^{∗} = ―8.38 to 0.21 or wherein ; b^{∗} = -4.88 to - 8.18;
iii. b^{∗} = 0.68a^{∗} - 8.32, wherein a^{∗} = 0.21 to 9.78 or wherein b^{∗} = -8.18 to - 1.67;
iv. b^{∗} = ―3.35a^{∗} + 31.10, wherein a^{∗} = 6.68 to 9.78 or wherein b^{∗} = 8.72 to - 1.67;
v. b^{∗} = ―0.48a^{∗} + 11.94, wherein a^{∗} = ―2.39 to 6.68 or wherein b^{∗} = 13.09 to 8.72; and
vi. b^{∗} = 0.89a^{∗} + 15.22, wherein a^{∗} = ―9.84 to - 2.39 or wherein b^{∗} = 6.46 to 13.09; and
wherein an L^{∗} of the color is 100 or less.

4. A packaging material according to any of the preceding claims wherein the printed portion comprises a text character (37) having a P/C Ratio of 3 or greater, wherein the P/C Ratio is the character height per spacing.

5. A packaging material according to any of the preceding claims wherein the printed portion comprise a text character (37) having a N/A Ratio of 6 or greater, wherein the N/A Ratio is the number per character area.

6. A packaging material according to any of the preceding claims wherein the three-dimensional structures comprise an average diameter of 100 micrometers to 400 micrometers.

7. A packaging material according to any of the preceding claims wherein the plurality of discrete three-dimensional projections comprise an average height of 50 micrometers to 400 micrometers.

8. A packaging material according to any of the preceding claims wherein textured zone comprises from 30 to 120 three-dimensional projections per linear inch.

9. A packaging material according to any of the preceding claims wherein the printed portion comprises a text character having a font size of 10 pt or less.

10. A packaging material according to any of the preceding claims wherein the substrate further comprises a flat zone (26).

11. A packaging material according to any of the preceding claims further comprising a second material layer (50) joined to the substrate.

12. A packaging material according to claim 11 wherein the printed portion is at least partially disposed on the second material layer.

13. A packaging material according to claims 11 or 12 wherein the second material layer is void of microtexture.

14. A packaging material according to any of claims 11-13 wherein the substrate comprises less than 1%, by weight of the substrate, of pigment.

15. A method of making the packaging material of any of claims 1-13 **characterized by** the steps of:
a. providing a substrate comprising a first surface, an opposing second surface, and a textured zone comprising a plurality of discrete three-dimensional projections comprising a sidewall extending outwardly from the first surface;
b. printing the first and/or the second opposing surface to create one or more printed portions, wherein at least some of the textured zone overlays at least some of at least one printed portion;
c. manipulating the textured zone in an area so that at least some of the plurality of discrete three-dimensional projections are physically altered to change the visible appearance of the printed portion in the area.

## Patentansprüche

1. Verpackungsmaterial (100), umfassend:
a. ein Substrat mit einer ersten Oberfläche (12) und einer entgegengesetzten zweiten Oberfläche (14), wobei wenigstens ein Abschnitt der ersten Oberfläche eine Außenfläche (115) des Verpackungsmaterials bildet;
b. einen bedruckten Abschnitt (30), der von der ersten Oberfläche aus sichtbar ist;
c. wobei die erste Oberfläche eine texturierte Zone (16) umfasst, die eine Vielzahl von separaten dreidimensionalen Vorsprüngen (18) umfasst;
d. wobei mindestens einige der Vielzahl von separaten dreidimensionalen Vorsprüngen eine Seitenwand (20), die sich von der ersten Oberfläche nach außen erstreckt, und ein distales Ende (24) umfassen;
e. wobei
es mindestens eine gewisse Überlappung zwischen der texturierten Zone und dem bedruckten Abschnitt gibt; **dadurch gekennzeichnet, dass:**
f. der bedruckte Abschnitt eine Farbe mit Koordinaten umfasst, die außerhalb einer CIELab-Farbgrenze liegen, die durch die folgenden Gleichungen beschrieben wird:
i.) b^{∗} = ―4,77a^{∗} - 42,21, worin a^{∗} = ―10,14 bis - 7,72 oder worin b^{∗} = 6,18 bis ― 5,39;
ii.) b^{∗} = ―0,32a^{∗} - 7,85, worin a^{∗} = ―7,72 bis 1,29 oder worin b^{∗} = ―5,39 bis -8,26;
iii.) b^{∗} = 0,82a^{∗} - 9,32, worin a^{∗} = 1,29 bis 9,36 oder worin b^{∗} = ―8,26 bis -1,64;
iv.) b^{∗} = ―4,08a^{∗} + 36,52, worin a^{∗} = 6,59 bis 9,36 oder worin b^{∗} = 9,63 bis -1,64;
v.) b^{∗} = ―0,44a^{∗} + 12,53, worin a^{∗} = ―2,19 bis 6,59 oder worin b^{∗} = 13,49 bis 9,63; und
vi.) b^{∗} = 0,92a^{∗} + 15,51, worin a^{∗} = ―10,14 bis ― 2,19 oder worin b^{∗} = 6,18 bis 13,49; und
wobei ein L* der Farbe 100 oder weniger ist.

2. Verpackungsmaterial nach Anspruch 1, wobei das Substrat eine Folie umfasst.

3. Verpackungsmaterial nach Anspruch 1 oder 2, wobei das Substrat auf die zweite Oberfläche gedruckt ist und wobei der bedruckte Abschnitt auf der zweiten Oberfläche eine Farbe mit Koordinaten umfasst, die außerhalb einer CIELab-Farbgrenze liegen, die durch die folgenden Gleichungen beschrieben wird:
i. b^{∗} = ―7,79a^{∗} - 70,16, worin a^{∗} = ―9,84 bis - 8,38 oder worin b^{∗} = 6,46 bis - 4,88;
ii. b^{∗} = ―0,38a^{∗} - 8,10, worin a^{∗} = ―8,38 bis 0,21 oder worin b^{∗} = ―4,88 bis -8,18;
iii. b^{∗} = 0,68a^{∗} - 8,32, worin a^{∗} = 0,21 bis 9,78 oder worin b^{∗} = ―8,18 bis -1,67;
iv. b^{∗} = ―3,35a^{∗} + 31,10, worin a^{∗} = 6,68 bis 9,78 oder worin b^{∗} = 8,72 bis -1,67;
v. b^{∗} = ―0,48a^{∗} + 11,94, worin a^{∗} = ―2,39 bis 6,68 oder worin b^{∗} = 13,09 bis 8,72; und
vi. b^{∗} = 0,89a^{∗} + 15,22, worin a^{∗} = ―9,84 bis - 2,39 oder worin b^{∗} = 6,46 bis 13,09, und
wobei ein L^{∗} der Farbe 100 oder weniger ist.

4. Verpackungsmaterial nach einem der vorstehenden Ansprüche, wobei der bedruckte Abschnitt ein Textzeichen (37) mit einem P/C-Verhältnis von 3 oder mehr umfasst, wobei das P/C-Verhältnis die Zeichenhöhe pro Abstand ist.

5. Verpackungsmaterial nach einem der vorstehenden Ansprüche, wobei der bedruckte Abschnitt ein Textzeichen (37) mit einem N/A-Verhältnis von 6 oder mehr umfasst, wobei das N/A-Verhältnis die Anzahl pro Zeichenfläche ist.

6. Verpackungsmaterial nach einem der vorstehenden Ansprüche, wobei die dreidimensionalen Strukturen einen durchschnittlichen Durchmesser von 100 Mikrometern bis 400 Mikrometern umfassen.

7. Verpackungsmaterial nach einem der vorstehenden Ansprüche, wobei die Vielzahl separater dreidimensionaler Vorsprünge eine durchschnittliche Höhe von 50 Mikrometern bis 400 Mikrometern umfasst.

8. Verpackungsmaterial nach einem der vorstehenden Ansprüche, wobei die texturierte Zone von 30 bis 120 dreidimensionale Vorsprünge pro linearem Zoll umfasst.

9. Verpackungsmaterial nach einem der vorstehenden Ansprüche, wobei der bedruckte Abschnitt ein Textzeichen mit einer Schriftgröße von 10 pt oder weniger umfasst.

10. Verpackungsmaterial nach einem der vorstehenden Ansprüche, wobei das Substrat ferner eine flache Zone (26) umfasst.

11. Verpackungsmaterial nach einem der vorstehenden Ansprüche, ferner umfassend eine zweite Materialschicht (50), die mit dem Substrat verbunden ist.

12. Verpackungsmaterial nach Anspruch 11, wobei der bedruckte Abschnitt mindestens teilweise auf der zweiten Materialschicht angeordnet ist.

13. Verpackungsmaterial nach Anspruch 11 oder 12, wobei die zweite Materialschicht frei von Mikrotextur ist.

14. Verpackungsmaterial nach einem der Ansprüche 11 bis 13, wobei das Substrat zu weniger als 1 Gew.-% des Substrats Pigment umfasst.

15. Verfahren zur Herstellung des Verpackungsmaterials nach einem der Ansprüche 1 bis 13, **gekennzeichnet durch** die Schritte:
a. Bereitstellen eines Substrats, das eine erste Oberfläche, eine entgegengesetzte zweite Oberfläche und eine texturierte Zone umfasst, die eine Vielzahl von separaten dreidimensionalen Vorsprüngen umfasst, die eine Seitenwand umfassen, die sich von der ersten Oberfläche nach außen erstreckt;
b. Bedrucken der ersten und/oder der zweiten entgegengesetzten Oberfläche, um einen oder mehrere bedruckte Abschnitte zu erzeugen, wobei mindestens ein Teil der texturierten Zone mindestens einen Teil des mindestens einen bedruckten Abschnitts überlagert;
c. Manipulieren der texturierten Zone in einem Bereich, sodass mindestens einige der Vielzahl von separaten dreidimensionalen Vorsprüngen physisch verändert werden, um das sichtbare Erscheinungsbild des bedruckten Abschnitts in dem Bereich zu verändern.

## Revendications

1. Matériau de conditionnement (100) comprenant :
a. un substrat ayant une première surface (12) et une deuxième surface opposée (14), dans lequel au moins une partie de la première surface forme une face extérieure (115) du matériau de conditionnement ;
b. une partie imprimée (30) qui est visible depuis la première surface ;
c. dans lequel la première surface comprend une zone texturée (16) comprenant une pluralité de saillies tridimensionnelles discrètes (18) ;
d. dans lequel au moins certaines parmi la pluralité de saillies tridimensionnelles discrètes comprennent une paroi latérale (20) s'étendant vers l'extérieur à partir de la première surface et une extrémité distale (24) ;
e. dans lequel il y a au moins un certain chevauchement entre la zone texturée et la partie imprimée ; **caractérisé en ce que :**
f. la partie imprimée comprend une couleur ayant des coordonnées qui sont à l'extérieur d'une limite de couleur CIELab décrite par les équations suivantes :
i.) b^{∗} = ―4,77a^{∗} - 42,21, dans lequel a^{∗} = ―10,14 à - 7,72 ou dans lequel b^{∗} = 6,18 à - 5,39 ;
ii.) b^{∗} = ―0,32a^{∗} - 7,85, dans lequel a^{∗} = ―7,72 à 1,29 ou dans lequel b^{∗} = ―5,39 à - 8,26 ;
iii.) b^{∗} = 0,82a^{∗} - 9,32, dans lequel a^{∗} = 1,29 à 9,36 ou dans lequel b^{∗} = ―8,26 à - 1,64 ;
iv.) b^{∗} = ―4,08a^{∗} + 36,52, dans lequel a^{∗} = 6,59 à 9,36 ou dans lequel b^{∗} = 9,63 à - 1,64 ;
v.) b^{∗} = ―0,44a^{∗} + 12,53, dans lequel a^{∗} = ―2,19 à 6,59 ou dans lequel b^{∗} = 13,49 à 9,63 ; et
vi.) b^{∗} = 0,92a^{∗} + 15,51, dans lequel a^{∗} = ―10,14 à - 2,19 ou dans lequel b^{∗} = 6,18 à 13,49 ; et
dans lequel un L* de la couleur vaut 100 ou moins.

2. Matériau de conditionnement selon la revendication 1 dans lequel le substrat comprend un film.

3. Matériau de conditionnement selon les revendications 1 ou 2 dans lequel le substrat est imprimé sur la deuxième surface, et dans lequel la partie imprimée sur la deuxième surface comprend une couleur ayant des coordonnées qui sont à l'extérieur d'une limite de couleur CIELab décrite par les équations suivantes :
i. b^{∗} = ―7,79a^{∗} - 70,16, dans lequel a^{∗} = ―9,84 à - 8,38 ou dans lequel b^{∗} = 6,46 à - 4,88 ;
ii. b^{∗} = ―0,38a^{∗} - 8,10, dans lequel a^{∗} = ―8,38 à 0,21 ou dans lequel ; b^{∗} = ―4,88 à - 8,18 ;
iii. b^{∗} = 0,68a^{∗} - 8,32, dans lequel a^{∗} = 0,21 à 9,78 ou dans lequel b^{∗} = -8,18 à - 1,67 ;
iv. b^{∗} = ―3,35a^{∗} + 31,10, dans lequel a^{∗} = 6,68 à 9,78 ou dans lequel b^{∗} = 8,72 à - 1,67 ;
v. b^{∗} = ―0,48a^{∗} + 11,94, dans lequel a^{∗} = ―2,39 à 6,68 ou dans lequel b^{∗} = 13,09 à 8,72 ; et
vi. b^{∗} = 0,89a^{∗} + 15,22, dans lequel a^{∗} = ―9,84 à - 2,39 ou dans lequel b^{∗} = 6,46 à 13,09 ; et
dans lequel un L* de la couleur vaut 100 ou moins.

4. Matériau de conditionnement selon l'une quelconque des revendications précédentes dans lequel la partie imprimée comprend un caractère de texte (37) ayant un rapport P/C de 3 ou plus, dans lequel le rapport P/C est la hauteur de caractère par espacement.

5. Matériau de conditionnement selon l'une quelconque des revendications précédentes dans lequel la partie imprimée comprend un caractère de texte (37) ayant un rapport N/A de 6 ou plus, dans lequel le rapport N/A est le nombre par superficie de caractère.

6. Matériau de conditionnement selon l'une quelconque des revendications précédentes dans lequel les structures tridimensionnelles comprennent un diamètre moyen de 100 micromètres à 400 micromètres.

7. Matériau de conditionnement selon l'une quelconque des revendications précédentes dans lequel la pluralité de saillies tridimensionnelles discrètes comprennent une hauteur moyenne de 50 micromètres à 400 micromètres.

8. Matériau de conditionnement selon l'une quelconque des revendications précédentes dans lequel une zone texturée comprend de 30 à 120 saillies tridimensionnelles par pouce linéaire.

9. Matériau de conditionnement selon l'une quelconque des revendications précédentes dans lequel la partie imprimée comprend un caractère de texte ayant une taille de police de 10 pt ou moins.

10. Matériau de conditionnement selon l'une quelconque des revendications précédentes dans lequel le substrat comprend en outre une zone plate (26).

11. Matériau de conditionnement selon l'une quelconque des revendications précédentes comprenant en outre une deuxième couche de matériau (50) jointe au substrat.

12. Matériau de conditionnement selon la revendication 11 dans lequel la partie imprimée est au moins partiellement disposée sur la deuxième couche de matériau.

13. Matériau de conditionnement selon les revendications 11 ou 12 dans lequel la deuxième couche de matériau est exempte de microtexture.

14. Matériau de conditionnement selon l'une quelconque des revendications 11 à 13 dans lequel le substrat comprend moins de 1 %, en poids du substrat, de pigment.

15. Procédé de fabrication du matériau de conditionnement selon l'une quelconque des revendications 1 à 13 **caractérisé par** les étapes consistant à :
a. fournir un substrat comprenant une première surface, une deuxième surface opposée, et une zone texturée comprenant une pluralité de saillies tridimensionnelles discrètes comprenant une paroi latérale s'étendant vers l'extérieur à partir de la première surface ;
b. imprimer la première surface et/ou la deuxième surface opposée pour créer une ou plusieurs parties imprimées, dans lequel au moins une partie de la zone texturée recouvre au moins une partie d'au moins une partie imprimée ;
c. manipuler la zone texturée dans une zone de sorte qu'au moins certaines parmi la pluralité de saillies tridimensionnelles discrètes sont physiquement modifiées pour changer l'apparence visible de la partie imprimée dans la zone.
